# EUROPEAN PATENT APPLICATION

(11) **EP 4 442 269 A1**
(43) Date of publication of application: **09.10.2024**
(21) Application number: 22900342.1
(22) Date of filing: 23.11.2022
(51) Int. Cl.: A61K 38/05, A61K 9/10, A61K 9/08, A61P 27/02, A61P 27/10

(54) **NOVEL USE OF ALANYL-GLUTAMINE AND OPHTHALMIC COMPOSITION COMPRISING ALANYL-GLUTAMINE**

(30) Priority: 01.12.2021 CN 202111450922
(71) Applicant: Seinda Pharmaceutical Guangzhou Corporation, Guangzhou, 510300 (CN)
(72) Inventor: HUANG, Jingfeng, HANGZHOU, Guangdong 510300 (CN); LIU, Yanan, Guangzhou, Guangdong 510300 (CN); DU, Tieliang, GUANGZHOU, Guangdong 510300 (CN)
(74) Representative: Isarpatent
(86) International application number: PCT/CN2022/133753
(87) International publication number: WO 2023/098537

(57) **Abstract**

A use of alanyl-glutamine in preparation for a drug for treating, relieving or alleviating ocular surface diseases and/or conditions of non-dry eyes. A use of alanyl-glutamine in preparation for a drug for relieving or alleviating visual fatigue. A use of alanyl-glutamine in preparation for a beauty product and health-care product for relieving or alleviating asthenopia.

## Description

### TECHNICAL FIELD

The present disclosure relates to the field of biotechnology and pharmacy, specifically to a novel use of alanyl-glutamine, and more specifically to use of alanyl-glutamine in the manufacture of a drug for treating, relieving or alleviating ocular surface diseases and/or conditions of non-dry eyes; use of alanyl-glutamine in the manufacture of a drug for treating, relieving or alleviating ocular surface diseases and/or conditions associated with corneal epithelial injury; use of alanyl-glutamine in the manufacture of a drug for treating, relieving or alleviating ocular surface diseases and/or conditions associated with keratopathy caused by diabetes; use of alanyl-glutamine in the manufacture of a drug for treating, relieving or alleviating ocular surface diseases and/or conditions associated with surgically related recurrent corneal epithelial erosion; use of alanyl-glutamine in the manufacture of a drug for treating, relieving or alleviating conjunctival congestion and/or other conditions associated with conjunctivitis (eye redness); use of alanyl-glutamine in the manufacture of a drug for treating, relieving or alleviating ocular surface diseases and/or conditions associated with meibomian gland dysfunction; use of alanyl-glutamine in the manufacture of a drug for treating, relieving or alleviating ocular surface diseases and/or conditions associated with asthenopia; use of alanyl-glutamine in the manufacture of a beauty product, a health-care product for relieving or alleviating asthenopia; and use of alanyl-glutamine in the manufacture of a beauty product, a health-care product for eyes. The present disclosure further relates to an ophthalmic composition comprising alanyl-glutamine and an ophthalmologically acceptable excipient, wherein the ophthalmic composition is in the form of eye drops, suspensions, eye ointments, emulsions, eye pads, eye patches, eye masks, eye creams, sprays, gels, injections or implants.

### BACKGROUND

As early as 1984, Nelson proposed the concept of ocular surface disease. Anatomically, the ocular surface includes the entire mucosal epithelial lining between the upper and lower eyelid margins. Histologically, this epithelial layer covers two major areas, i.e., the cornea and the conjunctiva. The main function of the ocular surface is to ensure clear vision when the eyes are open. It includes the cornea, conjunctival epithelium, and tear film. These three are closely related and influence each other. Changes in any of the three will lead to instability of the ocular surface. Therefore, from a narrow perspective, the ocular surface diseases refer only to diseases of the cornea and the conjunctival epithelium. From a relatively broader perspective, the ocular surface diseases should include superficial corneal and conjunctival diseases and diseases that can lead to abnormal tear film function, that is, "ocular surface diseases."

The transparency and functional integrity of the cornea depend on the normal structure and metabolism of layers of corneal tissue, and the corneal epithelial tissue located in the outermost layer undoubtedly plays a very important role in this regard. Various physical injuries, chemical injuries, mechanical injuries, pathogenic microorganisms, endocrine and immune factors can cause corneal epithelial injury. Corneal epithelium is a physical barrier against invasion by external pathogenic factors. The maintenance of its integrity depends on the tight junctions and anchoring junctions between epithelial cells, and between cells and the basement membrane, as well as the continuous self-renewal of the epithelial cells. Injury to the corneal epithelial layer can affect the junctions between cells, causing changes in the permeability and selectivity of the cell membrane, thereby affecting its barrier function. This can make the cornea vulnerable to external pathogenic factors, leading to inflammation, corneal opacity, and even blindness. Because of the destruction of the normal and complete epithelial barrier, persistent corneal epithelial injury can cause pathogenic microorganisms to easily penetrate the Bowman's membrane and invade the corneal parenchyma, causing corneal inflammation, corneal stroma opacity, collagen melting to form corneal ulcers, corneal perforation, and even eyeball loss, with serious consequences. Currently, traditional treatments for persistent corneal epithelial injury include: tear substitutes, corneal contact lenses, tarsorrhaphy, growth factors, autologous serum, etc., which are not effective.

Corneal ulcers are open sores that develop on the superficial cornea as a result of bacterial, viral, or fungal infections, mechanical injuries, or severe allergic diseases. Corneal ulcers are serious conditions that must be treated quickly to avoid problems with vision loss. Inflammation of the cornea, due to inflammation or injury, can lead to severe vision loss or even blindness. The deeper the corneal ulcer, the more serious the condition becomes and very deep ulcers can lead to scarring on the cornea, which subsequent blocks of light from entering the eye. Treatment usually involves antibiotics and antiviral or antifungal dugs. Steroid eye drops may also be given to reduce inflammation. However, there are no drugs that enhance wound closure and avoid scarring. In severe cases, when existing treatments have not helped, a corneal transplant may be needed to restore vision.

Diabetic keratopathy (DK) is one of the common complications of diabetes in the eye. Its main clinical manifestations include punctate keratitis, delayed corneal epithelial regeneration, recurrent corneal epithelial erosion, corneal hypoaesthesia, and corneal edema. Diabetic keratopathy was first proposed and named by Schultz. It is reported that 47% to 64% of diabetic patients will develop DK. DK is divided into two categories: primary and secondary DK. The primary DK refers to keratopathy caused by diabetes itself, and the secondary DK refers to corneal complications caused by eye surgery in diabetic patients.

Recurrent corneal erosion occurs when the corneal epithelium is injured (corneal rupture), and the adhesion between the regenerated epithelium and the basement membrane and Bowman's membrane is not tight enough, causing the possibility of tearing the incompletely healed corneal injury again once the eyes are opened after resting with the eyes closed, resulting in the repeated symptoms of corneal rupture (including eye stinging, redness, photodysphoria, lacrimation, inability to open the eyes, etc.), meanwhile which also allows bacteria to have opportunities to cause corneal infection and ulceration.

The conjunctiva is a transparent membrane covering the inner surface of the eyelids and the surface of the eye white of the front of the eyeball. Since most of the conjunctiva is in direct contact with the outside world, it is easily stimulated by infectious (such as bacteria, viruses, and chlamydia) and non-infectious factors (such as trauma, chemicals, and physical factors) in the surrounding environment, and the conjunctiva is rich in blood vessels and lymphatic tissue, and is easily sensitized by its own and external antigens. Conjunctivitis is inflammation or infection that occurs in the conjunctiva. Conjunctivitis is a common disease in ophthalmology, and is a general term for inflammatory responses such as edema and redness in the conjunctival tissue caused by external factors and the body's own factors. Although conjunctivitis itself does not affect vision seriously, it can cause vision damage when its inflammation spreads to cornea or causes complications. At present, clinical prevention and treatment of conjunctivitis mostly involves exogenous supplementation of artificial tears to relieve the clinical symptoms of conjunctivitis, or topical use of steroid hormones or non-steroidal anti-inflammatory drugs to reduce ocular surface inflammation. The above treatments can relieve some clinical symptoms of conjunctivitis to a certain extent, but they can only treat the symptoms rather than the root cause, and may even cause dependence on exogenous eye drops. In addition, the eye drops often contain preservatives for long-term storage to avoid eye infection caused by eye drop application, and long-term use of the eye drops containing the preservatives is not only unfavorable for treating conjunctivitis, but also causes eyes to feel dry, painful and itchy, thereby aggravating clinical symptoms of xerotic keratitis/conjunctivitis and causing injury to corneal epithelium in severe cases. Pinkeye is a common acute epidemic eye disease caused by bacterial or adenovirus infection. Its main characteristics are obvious conjunctival congestion and purulent or mucopurulent secretions. Bacteria can directly contact the conjunctiva through a variety of media and spread rapidly in public places, collective units such as kindergartens, schools and families, leading to epidemics. Typical symptoms of pinkeye are as follows: 75% of the patients have lacrimation, 75% of the patients have increased eye secretions, 75% of the patients are photophobic, 65% of the patients have ocular congestion and red eyes, and 65% of the patients have edema in the eyes. Patients consciously feel the eyes urticant like foreign body sensation, and have heaviness sense in the eyelids, photophobia and lacrimation and burning sensation when the eye itching is severe. Sometimes, temporary blurred vision is caused by the fact that secretions adhere to the pupil area on the corneal surface, and the vision can be recovered after flushing. A large amount of mucopurulent secretions are generated due to inflammation stimulation, patients can find that upper and lower eyelids are adhered together by the secretions when waking in the morning, and the photophobia, pain, hypopsia and other symptoms are obviously aggravated when the lesions invade into the cornea, and a small number of patients may have upper respiratory tract infection or other systemic symptoms at the same time. At present, the main methods to treat pinkeye are: using appropriate irrigants such as normal saline or 2% boric acid in water to flush the conjunctival sac, and wiping the eyelid margin with a sterile cotton swab; applying eye drops or eye ointments to the affected eyes, such as antibiotic eye drops, antiviral eye drops, and hormonal anti-inflammatory drugs. However, the above methods take a long treatment time and have serious side effects.

Ocular congestion refers to the redness of the whites of the eyes when the blood vessels of the bulbar conjunctiva and scleral tissue become dilated, congested, blood stasis, or hemorrhaged under certain circumstances. Since the blood supply sources of various parts of the eye are different, the congestion patterns of the eyes are also different, and the lesions reflected are also different. Therefore, ocular congestion is a common symptom shared by many eye diseases. Eyeball congestion is divided into two types: superficial and deep. The former is characterized by bright red and is called "conjunctival congestion"; The latter is characterized by dark red and is called "ciliary congestion". A combination of both is called "mixed congestion." Conjunctival congestion represents a primary or secondary disease of the conjunctiva or surrounding accessory organs. Ciliary congestion represents a disease of the eyeball itself, such as keratitis, scleritis, iridocyclitis, congestive glaucoma, etc. If the blood vessel itself is diseased or injured, bleeding can accumulate under the bulbar conjunctiva, which is called subconjunctival hemorrhage, which is also a type of ocular congestion. Eyeball congestion occurs when the capillaries on the surface of the eyeball become red and swollen and congested, often due to insufficient oxygen supply to the cornea or other tissues on the surface of the eye. Eye strain and incorrect eating habits, especially when consuming large amounts of ethanol, can also produce this phenomenon.

Meibomian gland dysfunction (MGD) is a chronic, diffuse meibomian gland disease characterized by obstruction of the terminal ducts of the meibomian glands and/or abnormal quality or quantity of meibum secretion, and it is a common ocular surface disease in clinic. The obstruction of the duct of meibomian can cause bacterial infection on the ocular surface, leading to inflammatory responses, eye irritation symptoms, corresponding changes in the cornea and conjunctiva, and in severe cases, decreasing vision, and causing poor prognosis. Mite infection is also a factor causing MGD, and the main treatments for MGD include eyelid warming, mechanical eyelid care, and pharmaceutical treatments such as antibiotics and anti-inflammatory agents. However, these treatments have certain limitations. Although eyelid warming can melt lipids, it cannot cope with the movement of keratinized substances, and it can also cause temporary visual degradation due to corneal deformation. Mechanical eyelid care can only provide partial and temporary relief of meibomian gland obstruction because the force required to remove the obstruction may be great and may cause significant pain to the patient. Topical use of antibiotics and corticosteroids to inhibit bacterial colonization and inflammation of the eyelid margin associated with MGD has been shown to be effective in relieving the symptoms and physical signs of MGD. However, on the one hand the success of this treatment may not be associated with altered meibum, and on the other hand drug intolerance and long-term treatment limit the clinical use of oral antibiotics.

Visual display terminal (VDT), also known as VDT syndrome, refers to a syndrome with a series of symptoms dominated by asthenopia such as eye fatigue, eye dryness, foreign body sensation in eyes, eyelid heaviness sensation, blurred vision, photophobia and lacrimation, eye swelling pain and ocular congestion after using a visual display terminal. According to statistics from the U.S. National Institute for Occupational Safety and Health, about 90% of people who spend an average of 3 hours or more per day on display devices will be affected by computer vision syndrome. According to the 42nd "Statistical Report on Internet Development in China", the scale of Chinese netizens reaches 802 million by June 2018, and the average weekly online time of Chinese netizens during the same period was up to 27.7 h, which is increased by 0.7 hour compared to the last year, and the popularization rate of the internet is increased to 57.7% from 55.8% of the end of last year. With the development of internet convenience, more and more online education has emerged. Most parents of students choose online classes for their children, which increases their children's opportunities to use electronic products. These netizens and students are prone to asthenopia, which can further induce problems such as myopia and MGD.

In summary, for ocular surface diseases and/or conditions associated with corneal epithelial injury, ocular surface diseases and/or disorders caused by diabetes, especially ocular surface diseases and/or conditions associated with keratopathy caused by diseases, a series of ocular surface diseases and/or conditions of non-dry eyes related to recurrent corneal epithelial erosion, especially surgically related recurrent corneal epithelial erosion, conjunctivitis (eye redness), ocular congestion, meibomian gland dysfunction, asthenopia and myopia and the like, there is an urgent need to develop an ophthalmic therapeutic drug and/or a beauty product, a health-care product, which has good therapeutic effect, can be used for a long time, and has no obvious topical or systemic toxic and side effects.

### SUMMARY

In order to solve the above-mentioned technical problems, the inventors conducted in-depth research and finally found a technical solution. Specifically, the present disclosure provides use of alanyl-glutamine in the manufacture of a drug for treating, relieving or alleviating ocular surface diseases and/or conditions of non-dry eyes.

The present disclosure further provides use of alanyl-glutamine in the manufacture of a drug for treating, relieving or alleviating ocular surface diseases and/or conditions associated with corneal epithelial injury.

The present disclosure provides use of alanyl-glutamine in the manufacture of a drug for treating, relieving or alleviating ocular surface diseases and/or conditions caused by diabetes.

The present disclosure provides use of alanyl-glutamine in the manufacture of a drug for treating, relieving or alleviating ocular surface diseases and/or conditions associated with keratopathy caused by diabetes.

The present disclosure provides use of alanyl-glutamine in the manufacture of a drug for treating, relieving or alleviating ocular surface diseases and/or conditions associated with recurrent corneal epithelial erosion.

The present disclosure provides use of alanyl-glutamine in the manufacture of a drug for treating, relieving or alleviating ocular surface diseases and/or conditions associated with surgically related recurrent corneal epithelial erosion.

The present disclosure provides use of alanyl-glutamine in the manufacture of a drug for treating, relieving or alleviating ocular surface diseases and/or conditions associated with ocular trauma and/or post-surgical tissue repair and healing.

The present disclosure further provides use of alanyl-glutamine in the manufacture of a drug for treating, relieving or alleviating ocular surface diseases and/or conditions associated with conjunctivitis (eye redness).

The present disclosure provides use of alanyl-glutamine in the manufacture of a drug for treating, relieving or alleviating ocular surface diseases and/or conditions associated with ocular congestion.

The present disclosure provides use of alanyl-glutamine in the manufacture of a drug for treating, relieving or alleviating ocular surface diseases and/or conditions associated with meibomian gland dysfunction.

The present disclosure further provides use of alanyl-glutamine in the manufacture of a drug for treating, relieving or alleviating ocular surface diseases and/or conditions associated with asthenopia.

The present disclosure provides use of alanyl-glutamine in the preparation for a drug for preventing myopia.

The present disclosure further provides use of alanyl-glutamine in the manufacture of a beauty product, a health-care product for relieving or alleviating asthenopia.

The present disclosure provides use of alanyl-glutamine in the manufacture of a beauty product, a health-care product for preventing myopia.

The present disclosure further provides use of alanyl-glutamine in the manufacture of a beauty product, a health-care product for eyes.

The present disclosure further provides an ophthalmic composition comprising alanyl-glutamine and an ophthalmologically acceptable excipient, wherein the ophthalmic composition is in the form of eye drops, suspensions, eye ointments, emulsions, eye pads, eye patches, eye masks, eye creams, sprays, gels, injections or implants.

The present disclosure provides a method for treating, relieving or alleviating ocular surface diseases and/or conditions of non-dry eyes, ocular surface diseases and/or conditions associated with corneal epithelial injury, ocular surface diseases and/or conditions caused by diabetes, ocular surface diseases and/or conditions associated with keratopathy caused by diabetes, ocular surface diseases and/or conditions associated with recurrent corneal epithelial erosion, ocular surface diseases and/or conditions associated with surgically related recurrent corneal epithelial erosion, ocular surface diseases and/or conditions associated with ocular trauma and/or post-surgical tissue repair and healing, ocular surface diseases and/or conditions associated with conjunctivitis, ocular surface diseases and/or conditions associated with ocular congestion, ocular surface diseases and/or conditions associated with meibomian gland dysfunction, ocular surface diseases and/or conditions associated with asthenopia, myopia, the method comprises administering to a subject a therapeutically effective amount of alanyl-glutamine.

The present disclosure further provides a method for treating, relieving or alleviating ocular surface diseases and/or conditions of non-dry eyes, ocular surface diseases and/or conditions associated with corneal epithelial injury, ocular surface diseases and/or conditions caused by diabetes, ocular surface diseases and/or conditions associated with keratopathy caused by diabetes, ocular surface diseases and/or conditions associated with recurrent corneal epithelial erosion, ocular surface diseases and/or conditions associated with surgically related recurrent corneal epithelial erosion, ocular surface diseases and/or conditions associated with ocular trauma and/or post-surgical tissue repair and healing, ocular surface diseases and/or conditions associated with conjunctivitis, ocular surface diseases and/or conditions associated with ocular congestion, ocular surface diseases and/or conditions associated with meibomian gland dysfunction, ocular surface diseases and/or conditions associated with asthenopia, myopia, the method comprises administering to a subject a therapeutically effective amount of the ophthalmic composition provided by the present disclosure.

### DETAILED DESCRIPTION OF EMBODIMENTS

Unless defined otherwise, all technical and scientific terms used herein have the same meanings as commonly understood by one of ordinary skill in the field to which the present disclosure belongs.

As used herein, the expression "A and/or B" includes 3 cases: (1) A; (2) B; and (3) A and B.

As used herein, reference to alanyl-glutamine encompasses alanyl-glutamine itself and pharmaceutically acceptable salts thereof unless otherwise stated or contradicted by context.

As used herein, the expression "pharmaceutically acceptable salt" includes acid addition salts formed with pharmaceutically acceptable acids, as well as salts formed with pharmaceutically acceptable bases. The pharmaceutically acceptable acids include inorganic acids, such as hydrochloric acid, hydrobromic acid, sulfuric acid, phosphoric acid, nitric acid, etc., and organic acids, such as acetic acid, citric acid, citric acid, fumaric acid, malic acid, etc. The salts formed with the pharmaceutically acceptable bases include, but are not limited to, ammonium salts, sodium salts, potassium salts, magnesium salts, calcium salts, etc.

The inventor of the present invention unexpectedly discovered that the ophthalmic solutions, the ophthalmic gels and the ophthalmic patches and masks containing alanyl-glutamine can effectively alleviate meibomian gland dysfunction, treat conjunctivitis (eye redness), treat corneal ulcer, and significantly relieve asthenopia and prevent myopia, thus indicating the use of alanyl-glutamine in drugs for treating, relieving or alleviating ocular surface diseases and/or conditions of non-dry eyes, ocular surface diseases and/or conditions associated with corneal epithelial injury, ocular surface diseases and/or conditions caused by diabetes, especially ocular surface diseases and/or conditions associated with keratopathy caused by diabetes, treating, relieving or alleviating ocular surface diseases and/or conditions associated with recurrent corneal epithelial erosion, especially ocular surface diseases and/or conditions associated with surgically related recurrent corneal epithelial erosion, treating, relieving or alleviating ocular surface diseases and/or conditions associated with ocular trauma and/or post-surgical tissue repair and healing, treating, relieving or alleviating ocular surface diseases and/or conditions associated with conjunctivitis(eye redness), treating, relieving or alleviating ocular surface diseases and/or conditions associated with ocular congestion, treating, relieving or alleviating ocular surface diseases and/or conditions associated with meibomian gland dysfunction, ocular surface diseases and/or conditions associated with asthenopia and for preventing myopia.

According to an embodiment of the present disclosure, the present disclosure could provide use of alanyl-glutamine in the manufacture of a drug for treating, relieving or alleviating ocular surface diseases and/or conditions of non-dry eyes.

According to an embodiment of the present disclosure, the present disclosure could provide use of alanyl-glutamine in the manufacture of a drug for treating, relieving or alleviating ocular surface diseases and/or conditions associated with corneal epithelial injury.

In some embodiments, "ocular surface diseases and/or conditions associated with corneal epithelial injury" include, but not limited to, keratitis, corneal dystrophy, mechanical corneal epithelial injury, recurrent corneal epithelial erosion, entropion, trichiasis; blepharitis, meibomian gland dysfunction, hypophasis, band-shaped degeneration of cornea, Salzmann nodular corneal degeneration, post-contact lens corneal epithelial injury, drug related corneal epithelial injury, surgically related corneal epithelial injury, Steven-Johnson syndrome, Sjögren syndrome, cicatricial pemphigoid and the like caused by various factors.

According to an embodiment of the present disclosure, the present disclosure could provide use of alanyl-glutamine in the manufacture of a drug for treating, relieving or alleviating ocular surface diseases and/or conditions caused by diabetes.

According to an embodiment of the present disclosure, the present disclosure could provide use of alanyl-glutamine in the manufacture of a drug for treating, relieving or alleviating ocular surface diseases and/or conditions associated with keratopathy caused by diabetes.

In some embodiments, the "ocular surface diseases and/or conditions associated with keratopathy caused by diabetes" include, but not limited to, superficial punctate keratitis, delayed corneal epithelial regeneration, recurrent corneal epithelial erosion, persistent corneal epithelial loss, chronic epithelitis, superficial corneal ulcer, filamentous keratitis, sterile corneal ulcer, Descemet's membrane folds, corneal hypoaesthesia, and corneal edema.

According to an embodiment of the present disclosure, the present disclosure could provide use of alanyl-glutamine in the manufacture of a drug for treating, relieving or alleviating ocular surface diseases and/or conditions associated with recurrent corneal epithelial erosion.

In some embodiments of the present disclosure, the ocular surface diseases and/or conditions associated with recurrent corneal epithelial erosion may be surgically related or non-surgically related.

According to an embodiment of the present disclosure, the present disclosure could provide use of alanyl-glutamine in the manufacture of a drug for treating, relieving or alleviating ocular surface diseases and/or conditions associated with surgically related recurrent corneal epithelial erosion.

In some embodiments, "ocular surface diseases and/or conditions associated with surgically related recurrent corneal epithelial erosion" include, but not limited to, superficial punctate keratitis, delayed corneal epithelial regeneration, recurrent corneal epithelial erosion, persistent corneal epithelial loss, chronic epithelitis, superficial corneal ulcer, filamentous keratitis, sterile corneal ulcer, Descemet's membrane folds, corneal hypoaesthesia, and corneal edema.

According to an embodiment of the present disclosure, the present disclosure could provide use of alanyl-glutamine in the manufacture of a drug for treating, relieving or alleviating ocular surface diseases and/or conditions associated with ocular trauma and/or post-surgical tissue repair and healing.

In some embodiments, "ocular surface diseases and/or conditions associated with ocular trauma and/or post-surgical tissue repair and healing" include, but not limited to, ocular tissue injury and ocular foreign body sensation after cataract surgery, ocular tissue injury and ocular foreign body sensation after glaucoma surgery, ocular trauma and/or post-surgical ocular wounds, injury caused by mechanical ocular injury, injury caused by intraocular foreign body, injury caused by ocular chemical burn, injury caused by ocular thermal burn, injury caused by radiation induced ocular injury, injury caused by light injury, injury caused by ionizing radiation and microwaves, injury caused by eye electric shock and stress-induced ocular injury.

According to an embodiment of the present disclosure, the present disclosure could provide use of alanyl-glutamine in the manufacture of a drug for treating, relieving or alleviating ocular surface diseases and/or conditions associated with conjunctivitis (eye redness).

In some embodiments, "ocular surface diseases and/or conditions associated with conjunctivitis (eye redness)" include, but not limited to, bacterial conjunctivitis, viral conjunctivitis, Chlamydia trachomatis conjunctivitis, inclusion conjunctivitis, vernal keratoconjunctivitis, allergic conjunctivitis, seasonal allergic conjunctivitis, macropapillary conjunctivitis, phlyctenular kerato conjunctivitis, autoimmune conjunctivitis, conjunctival tumors, pterygium.

According to an embodiment of the present disclosure, the present disclosure could provide use of alanyl-glutamine in the manufacture of a drug for treating, relieving or alleviating ocular surface diseases and/or conditions associated with ocular congestion.

In some embodiments, "ocular surface diseases and/or conditions associated with ocular congestion" include, but not limited to, bacterial conjunctivitis, viral conjunctivitis, Chlamydia trachomatis conjunctivitis, inclusion conjunctivitis, vernal keratoconjunctivitis, allergic conjunctivitis, seasonal allergic conjunctivitis, acute iridocyclitis, keratitis, subconjunctival hemorrhage, macropapillary conjunctivitis, phlyctenular kerato conjunctivitis, autoimmune conjunctivitis, conjunctival tumors, pterygium, congestion caused by ocular surgery such as cataract and glaucoma surgery, and laser myopia surgery.

According to an embodiment of the present disclosure, the present disclosure could provide use of alanyl-glutamine in the manufacture of a drug for treating, relieving or alleviating ocular surface diseases and/or conditions associated with meibomian gland dysfunction.

In some embodiments, "ocular surface diseases and/or conditions associated with meibomian gland dysfunction" include, but are not limited to, blepharitis, meibomian gland dysfunction, and conjunctival congestion due to meibomian gland dysfunction.

According to an embodiment of the present disclosure, the present disclosure could provide use of alanyl-glutamine in the manufacture of a drug for treating, relieving or alleviating ocular surface diseases and/or conditions associated with asthenopia.

In some embodiments, "ocular surface diseases and/or conditions associated with asthenopia" include, but are not limited to, conjunctivitis, meibomian gland dysfunction, blepharitis, conjunctivitis, or ptosis.

According to an embodiment of the present disclosure, the present disclosure could provide use of alanyl-glutamine in the preparation of a drug for preventing myopia.

According to an embodiment of the present disclosure, the present disclosure could provide use of alanyl-glutamine in the manufacture of a beauty product, a health-care product for relieving or alleviating asthenopia.

According to an embodiment of the present disclosure, the present disclosure could provide use of alanyl-glutamine in the manufacture of a beauty product, a health-care product for preventing myopia.

As used herein, "beauty product" could take the form of, including, but not limited to, eye creams, eye gels, eye sprays, eye patches, eye pads, eye masks, and the like.

As used herein, "health-care product" could include, but not limited to, eye drops, collyria, eye gels, eye sprays, eye patches, eye pads, eye masks and the like of "Disinfection Product Number", "Health Product Number" and "Medical Device Product Number".

According to an embodiment of the present disclosure, the present disclosure could provide use of alanyl-glutamine in the manufacture of a beauty product, a health-care product for eyes.

According to an embodiment of the present disclosure, the present disclosure could provide an ophthalmic composition comprising alanyl-glutamine and an ophthalmologically acceptable excipient, wherein the ophthalmic composition is in the form of eye drops, suspensions, eye ointments, emulsions, eye pads, eye patches, eye masks, eye creams, sprays, gels, injections or implants.

In some embodiments, the ophthalmic composition may be a pharmaceutical product, a beauty product or a health-care product.

In some embodiments, the eye drops can be ophthalmic solutions or ophthalmic suspensions.

In some embodiments, the ophthalmic composition is in an isotonic or hypotonic form.

As used herein, "isotonic" refers to that the osmotic pressure of the ophthalmic pharmaceutical composition is the same or substantially the same as the osmotic pressure of the ocular surface epithelial cells. As used herein, "hypotonic" refers to that the osmotic pressure of the ophthalmic pharmaceutical composition is less than the osmotic pressure of the ocular surface epithelial cells.

In some embodiments, the concentration of the alanyl-glutamine in the ophthalmic composition is 0.05% w/w to 10% w/w.

In some embodiments, the concentration of the alanyl-glutamine in the ophthalmic composition is 0.05% w/w to 9% w/w, 0.05% w/w to 8% w/w, 0.05% w/w to 7%w/w, 0.05%w/w to 6%w/w, 0.05%w/w to 5%w/w, 0.05%w/w to 4%w/w, 0.05%w/w to 3%w/w, 0.05%w/w to 2%w/w, 0.05%w/w to 1%w/w or 0.05%w/w to 0.1%w/w.

In some embodiments, the ophthalmologically acceptable excipient includes one or more of an osmotic pressure regulator, a bacteriostatic agent, a viscosifier, a pH regulator, a stabilizer, a surfactant, a humectant, etc.

In some embodiments, the osmotic pressure regulator is selected from one or more of laurocapram, sodium chloride, potassium chloride, boric acid, borax, sodium sulfate, potassium sulfate, sodium nitrate, potassium nitrate, sodium acetate, mannitol, glycerol, propylene glycol, sorbitol, 2-(4-n-octylphenylethyl)-2-aminopropylene glycol hydrochloride, glucose, sucrose, fructose and lactose. In some embodiments, the osmotic pressure regulator is laurocapram, boric acid, glycerin, propylene glycol, and/or sodium chloride.

In some embodiments, the bacteriostatic agent is selected from one or more of benzalkonium chloride, benzalkonium bromide, quaternium salt-73, chlorhexidine acetate, chlorhexidine glucose, trichlorotert-butanol, phenoxyethanol, methyl hydroxybenzoate, ethyl hydroxybenzoate and propyl hydroxybenzoate. In some embodiments, the bacteriostatic agent is benzalkonium chloride, quaternium-73, phenoxyethanol, and/or ethyl hydroxybenzoate.

In some embodiments, the viscosifier is selected from one or more of sodium hyaluronate, triethanolamine, Carbopol, sodium carboxymethyl cellulose, methylcellulose, polyethylene glycol, polyvinyl alcohol and povidone. In some embodiments, the viscosifier is sodium hyaluronate, Carbopol 940, and/or triethanolamine.

In some embodiments, the pH regulator is selected from one or more of sodium dihydrogen phosphate, disodium hydrogen phosphate, potassium dihydrogen phosphate, dipotassium hydrogen phosphate, boric acid, borax, acetic acid, sodium acetate, citric acid, sodium citrate, tartaric acid, sodium tartrate, sodium carbonate, potassium carbonate, sodium bicarbonate, potassium bicarbonate, sodium hydroxide, potassium hydroxide, hydrochloric acid and phosphoric acid. In some embodiments, the pH regulator is selected from one or more of sodium carbonate, sodium bicarbonate, and sodium hydroxide. In some embodiments, the pH regulator is sodium hydroxide.

In some embodiments, the stabilizer is selected from one or more of disodium edetate, calcium disodium edetate, dipotassium edetate, diamine edetate, 2,3-dimercapto-1-propanesulfonic acid, dimercaptosuccinic acid, dimercaprol, deferoxamine mesylate, α-lipoic acid, nitrilotriacetate, penicillamine, ethylene glycol dimethacrylate, sodium oleate, anhydrous sodium sulfite, sodium ascorbate, deferoxamine, malic acid, citric acid, succinic acid, and sodium salts, calcium salts and magnesium salts of malic acid, citric acid and succinic acid. In some embodiments, the stabilizer is disodium edetate.

In some embodiments, the surfactant is selected from one or more of poloxamer, sodium dodecyl sulfate, Tween-20, Tween-40, Tween-60, Tween-65, Tween-80, Tween-85, lecithin, polyoxyethylene castor oil, polyoxyethylene hydrogenated castor oil, polyvinylpyrrolidone, polyethylene glycol and polyethylene glycol 15-hydroxystearate. In some embodiments, the surfactant is selected from Tween-80.

In some embodiments, the humectant is selected from one or more of glycerol, butanediol, polyethylene glycol, propylene glycol, hexanediol, xylitol, polypropylene glycol, sorbitol, sodium pyrrolidone carboxylate, sodium lactate, urea, hydroxyethyl urea, methyl glucose polyether, chitin derivative, trehalose, tremella polysaccharide, sodium hyaluronate, panthenol, ceramide, natural plant extract, etc.

According to an embodiment of the present disclosure, the present disclosure could provide a method of treating, relieving or alleviating ocular surface diseases and/or conditions of non-dry eyes, ocular surface diseases and/or conditions associated with corneal epithelial injury, ocular surface diseases and/or conditions caused by diabetes, ocular surface diseases and/or conditions associated with keratopathy caused by diabetes, ocular surface diseases and/or conditions associated with recurrent corneal epithelial erosion, ocular surface diseases and/or conditions associated with surgically related recurrent corneal epithelial erosion, ocular surface diseases and/or conditions associated with ocular trauma and/or post-surgical tissue repair and healing, ocular surface diseases and/or conditions associated with conjunctivitis, ocular surface diseases and/or conditions associated with ocular congestion, ocular surface diseases and/or conditions associated with meibomian gland dysfunction, ocular surface diseases and/or conditions associated with asthenopia, myopia, the method comprises administering to a subject a therapeutically effective amount of alanyl-glutamine.

As used herein, the expression "subject" refers to mammals, primates (e.g., humans, male or female), dogs, rabbits, guinea pigs, rats, mice, and the like. In some embodiments, the subject is a primate. In some embodiments, the subject is a human.

As used herein, the expression "a therapeutically effective amount" refers to an amount of the alanyl-glutamine or the ophthalmic composition of the present disclosure that is capable of eliciting a biological or medical response in a subject, such as an amount that treats, relieves, or alleviates diseases and/or conditions.

According to an embodiment of the present disclosure, the present disclosures could provide a method of treating, relieving or alleviating ocular surface diseases and/or conditions of non-dry eyes, the method comprises administering to a subject a therapeutically effective amount of alanyl-glutamine.

According to an embodiment of the present disclosure, the present disclosures could provide a method of treating, relieving or alleviating ocular surface diseases and/or conditions associated with corneal epithelial injury, the method comprises administering to a subject a therapeutically effective amount of alanyl-glutamine.

According to an embodiment of the present disclosure, the present disclosures could provide a method of treating, relieving or alleviating ocular surface diseases and/or conditions caused by diabetes, the method comprises administering to a subject a therapeutically effective amount of alanyl-glutamine.

According to an embodiment of the present disclosure, the present disclosures could provide a method of treating, relieving or alleviating ocular surface diseases and/or conditions associated with keratopathy caused by diabetes, the method comprises administering to a subject a therapeutically effective amount of alanyl-glutamine.

According to an embodiment of the present disclosure, the present disclosures could provide a method of treating, relieving or alleviating ocular surface diseases and/or conditions associated with recurrent corneal epithelial erosion, the method comprises administering to a subject a therapeutically effective amount of alanyl-glutamine.

According to an embodiment of the present disclosure, the present disclosures could provide a method of treating, relieving or alleviating ocular surface diseases and/or conditions associated with surgically related recurrent corneal epithelial erosion, the method comprises administering to a subject a therapeutically effective amount of alanyl-glutamine.

According to an embodiment of the present disclosure, the present disclosures could provide a method of treating, relieving or alleviating ocular surface diseases and/or conditions associated with ocular trauma and/or post-surgical tissue repair and healing, the method comprises administering to a subject a therapeutically effective amount of alanyl-glutamine.

According to an embodiment of the present disclosure, the present disclosures could provide a method of treating, relieving or alleviating ocular surface diseases and/or conditions associated with conjunctivitis, the method comprises administering to a subject a therapeutically effective amount of alanyl-glutamine.

According to an embodiment of the present disclosure, the present disclosures could provide a method of treating, relieving or alleviating ocular surface diseases and/or conditions associated with ocular congestion, the method comprises administering to a subject a therapeutically effective amount of alanyl-glutamine.

According to an embodiment of the present disclosure, the present disclosures could provide a method of treating, relieving or alleviating ocular surface diseases and/or conditions associated with meibomian gland dysfunction, the method comprises administering to a subject a therapeutically effective amount of alanyl-glutamine.

According to an embodiment of the present disclosure, the present disclosures could provide a method of treating, relieving or alleviating ocular surface diseases and/or conditions associated with asthenopia, the method comprises administering to a subject a therapeutically effective amount of alanyl-glutamine.

According to an embodiment of the present disclosure, the present disclosures could provide a method of preventing myopia, the method comprises administering to a subject a therapeutically effective amount of alanyl-glutamine.

According to an embodiment of the present disclosure, the present disclosures could provide a method of treating, relieving or alleviating ocular surface diseases and/or conditions of non-dry eyes, ocular surface diseases and/or conditions associated with corneal epithelial injury, ocular surface diseases and/or conditions caused by diabetes, ocular surface diseases and/or conditions associated with keratopathy caused by diabetes, ocular surface diseases and/or conditions associated with recurrent corneal epithelial erosion, ocular surface diseases and/or conditions associated with surgically related recurrent corneal epithelial erosion, ocular surface diseases and/or conditions associated with ocular trauma and/or post-surgical tissue repair and healing, ocular surface diseases and/or conditions associated with conjunctivitis, ocular surface diseases and/or conditions associated with ocular congestion, ocular surface diseases and/or conditions associated with meibomian gland dysfunction, ocular surface diseases and/or conditions associated with asthenopia, myopia, the method comprises administering to a subject the ophthalmic composition of the present disclosure.

According to an embodiment of the present disclosure, the present disclosures could provide a method of treating, relieving or alleviating ocular surface diseases and/or conditions of non-dry eyes, the method comprises administering to a subject a therapeutically effective amount of the ophthalmic composition of the present disclosure .

According to an embodiment of the present disclosure, the present disclosures could provide a method of treating, relieving or alleviating ocular surface diseases and/or conditions associated with corneal epithelial injury, the method comprises administering to a subject a therapeutically effective amount of the ophthalmic composition of the present disclosure.

According to an embodiment of the present disclosure, the present disclosures could provide a method of treating, relieving or alleviating ocular surface diseases and/or conditions caused by diabetes, the method comprises administering to a subject a therapeutically effective amount of the ophthalmic composition of the present disclosure.

According to an embodiment of the present disclosure, the present disclosures could provide a method of treating, relieving or alleviating ocular surface diseases and/or conditions associated with keratopathy caused by diabetes, the method comprises administering to a subject a therapeutically effective amount of the ophthalmic composition of the present disclosure.

According to an embodiment of the present disclosure, the present disclosures could provide a method of treating, relieving or alleviating ocular surface diseases and/or conditions associated with recurrent corneal epithelial erosion, the method comprises administering to a subject a therapeutically effective amount of the ophthalmic composition of the present disclosure.

According to an embodiment of the present disclosure, the present disclosures could provide a method of treating, relieving or alleviating ocular surface diseases and/or conditions associated with surgically related recurrent corneal epithelial erosion, the method comprises administering to a subject a therapeutically effective amount of the ophthalmic composition of the present disclosure.

According to an embodiment of the present disclosure, the present disclosures could provide a method of treating, relieving or alleviating ocular surface diseases and/or conditions associated with ocular trauma and/or post-surgical tissue repair and healing, the method comprises administering to a subject a therapeutically effective amount of the ophthalmic composition of the present disclosure .

According to an embodiment of the present disclosure, the present disclosures could provide a method of treating, relieving or alleviating ocular surface diseases and/or conditions associated with conjunctivitis, the method comprises administering to a subject a therapeutically effective amount of the ophthalmic composition of the present disclosure.

According to an embodiment of the present disclosure, the present disclosures could provide a method of treating, relieving or alleviating ocular surface diseases and/or conditions associated with ocular congestion, the method comprises administering to a subject a therapeutically effective amount of the ophthalmic composition of the present disclosure.

According to an embodiment of the present disclosure, the present disclosures could provide a method of treating, relieving or alleviating ocular surface diseases and/or conditions associated with meibomian gland dysfunction, the method comprises administering to a subject a therapeutically effective amount of the ophthalmic composition of the present disclosure.

According to an embodiment of the present disclosure, the present disclosures could provide is a method of treating, relieving or alleviating ocular surface diseases and/or conditions associated with asthenopia, the method comprises administering to a subject a therapeutically effective amount of the ophthalmic composition of the present disclosure.

According to an embodiment of the present disclosure, the present disclosures could provide a method of preventing myopia, the method comprises administering to a subject a therapeutically effective dose of the ophthalmic composition of the present disclosur.

The technical solution of the present disclosure will be more clearly and explicitly described by way of illustration in combination with examples. It should be understood that these examples are only for illustrative purposes and not intended to limit the protection scope of the present disclosure. The protection scope of the present disclosure is only defined by the claims.

### EXAMPLES

Unless otherwise stated, the reagents and instruments used in the following examples are conventional products that are commercially available. Unless otherwise stated, experiments are performed under conventional conditions or conditions recommended by the manufacturer. The "L-alanyl-L-glutamine", i.e., alanyl-glutamine itself used in the examples was available from Hubei Bioche Pharmaceutical Technology Co., Ltd. The 0.25% chloramphenicol eye drops used in the examples were available from Shandong Bausch & Lamb Freda Pharmaceutical Co., Ltd., the 0.1% diclofenac sodium eye drops were available from Shenyang Xingqi Eye Medicine Co., Ltd., the 0.1% pranoprofen eye drops were available from Guangdong Zhongsheng Pharmaceutical Co., Ltd., the sodium hyaluronate eye drops were available from Zhejiang Jianfeng Pharmaceutical Co., Ltd., Zhenshiming eye drops were available from Zhenshiming Pharmaceutical Co., Ltd., and the polyvinyl alcohol eye drops were available from Hubei Yuanda Tiantianming Pharmaceutical Co., Ltd.

### Example 1: Preparation of ophthalmic solution, ophthalmic cream and ophthalmic gel containing alanyl-glutamine

The ophthalmic solution, the ophthalmic cream, and the ophthalmic gel containing alanyl-glutamine were prepared according to the following formulation and preparation method:

**Table 1. Formulation of the ophthalmic solution**

| Ingredient | Percent (w/w) | |
|---|---|---|
| L-alanyl-L-glutamine | 0.05% | 10.0% |
| Sodium chloride | 0.40% | 0.00% |
| Boric acid | 1.0% | 1.0% |
| Benzalkonium chloride | 0.01% | 0.01% |
| Disodium edetate | 0.1% | 0.1% |
| Water for injection | 98.44% | 88.89% |

Preparation method of ophthalmic solution:
(1) Boric acid, sodium chloride, benzalkonium chloride, and disodium edetate were weighed in the proportions described in Table 1, and 80% water for injection was added, and dissolved under stirring.
(2) L-alanyl-L-glutamine was added in the proportion described in Table 1, and dissolved under stirring. The pH was adjusted to 6.0 with 0.5 mol/L sodium hydroxide under stirring.
(3) The remaining volume was compensated with water for injection, the obtained mixture was subjected to two-stage filtration through sterile filters of 0.45 µm and 0.22 µm, respectively, and then filled into eye drop bottles.

The solution excipient was prepared according to the above formulation and method. The only difference between the solution excipient and the ophthalmic solution was that: L-alanyl-L-glutamine was replaced with an equal amount of water.

**Table 2. Formulation of the ophthalmic cream**

| Ingredient | | Percent (w/w) | |
|---|---|---|---|
| L-alanyl-L-glutamine | | 0.05% | 10.0% |
| Oil phase | White vaseline | 5.0% | 5.0% |
| | Liquid paraffin | 12.0% | 12.0% |
| | Stearic acid | 10.0% | 10.0% |
| | Glyceryl monostearate | 5.0% | 5.0% |
| Aqueous phase | Glycerin | 10% | 10% |
| | Sodium dodecyl sulfate | 0.2% | 0.2% |
| | Methyl hydroxybenzoate | 0.2% | 0.2% |
| | Sodium hyaluronate | 0.1% | 0.1% |
| | Disodium edetate | 0.05% | 0.05% |
| | Purified water | 57.4% | 47.45% |

Preparation method of ophthalmic cream:
(1) White vaseline, liquid paraffin, stearic acid and glyceryl monostearate were weighed in the proportions described in Table 2, added to an oil phase tank, heated to 75-85°C to melt, stirred evenly, and used as the oil phase.
(2) Purified water, methyl hydroxybenzoate, sodium lauryl sulfate, glycerin, sodium hyaluronate, and disodium edetate were weighed in the proportions described in Table 2, added to an aqueous phase tank, heated to 75-85°C, stirred and dissolved, and used as the aqueous phase.
(3) L-alanyl-L-glutamine was added to the aqueous phase in the proportion described in Table 2 and dissolved.
(4) The oil phase liquid was added in an emulsification tank, and stirring was started, and then the aqueous phase liquid was added in the emulsification tank. The mixture was emulsified at 65-75°C for 20-30 min, and cooled down to below 35°C, stirring was stopped, and a cream was obtained.

The cream excipient was prepared according to the above formulation and method. The only difference between the cream excipient and the ophthalmic cream was that: L-alanyl-L-glutamine was replaced with an equal amount of water.

**Table 3. Formulation of the ophthalmic gel**

| Ingredient | Weight (g) | |
|---|---|---|
| L-alanyl-L-glutamine | 0.05g | 10.0g |
| Carbopol 940 | 1g | 1g |
| Glycerin | 10g | 10g |
| Ethanol | 5g | 5g |
| Propylene glycol | 5g | 5g |
| Tween-80 | 1g | 1g |
| Triethanolamine | 1g | 1g |
| Ethyl hydroxybenzoate | 0.3g | 0.3g |
| Purified water | 76.65g | 66.7g |

Preparation method of ophthalmic gel:
(1) L-alanyl-L-glutamine was dissolved in 50 ml of purified water to obtain an aqueous solution of L-alanyl-L-glutamine.
(2) Carbopol 940 and glycerin was thoroughly mixed, and added to the aqueous solution of L-alanyl-L-glutamine obtained in (1), and Carbopol was fully swollen.
(3) Ethyl hydroxybenzoate was dissolved in ethanol, mixed with propylene glycol, stirred, and slowly added to the solution in (2).
(4) Tween-80 and triethanolamine was dissolved in the remaining volume of purified water, added to the solution in (3) under stirring, and stirred until uniform to obtain a gel.

The gel excipient was prepared according to the above formulation and method. The only difference between the cream gel and the ophthalmic gel was that: L-alanyl-L-glutamine was replaced with an equal amount of water.

### Example 2: Irritation test

### (1) Skin irritation test

36 healthy New Zealand rabbits were randomly divided into 6 groups, 6 rabbits/group, half male and half female, and used for skin irritation experiments of single-dose and multiple-dose, each experiment was divided into 2 groups, i.e., intact skin group and injured skin group, and all comparisons were performed using the homobody left-right self-control method. Hair removal was performed on the backs of New Zealand rabbits using 8.0% sodium sulfide aqueous solution and on two sites (with a range of approximately 3.0 cm × 3.0 cm) of the backs of each rabbit, and New Zealand rabbits with erythema, edema or injury in the hair removal areas were eliminated. An injured skin model was established after 24 h of hair removal: First the hair removal areas were washed with warm water and disinfected with iodophor, then a sterile needle was used to lightly mark the "#" symbol thereon and skin injury was caused to the degree of slight intensive bleeding on the skin; A intact skin model was established: the skin area that was intact, not injured and not abnormal was selected.

10% ophthalmic solution and solution excipient, 10% ophthalmic cream and cream excipient, 10% ophthalmic gel and gel excipient were applied evenly to the hair removal areas of different New Zealand rabbit (the injured skin group and the intact skin group), and the surface was covered with thin gauze, bandaged and fixed with non-irritating breathable non-woven tape. Single-dose skin irritation experiment (administration once) and multiple-dose skin irritation experiment (continuous administration for 14 days, once a day). Specific steps: The test substances were in contact with the skin for 24 h, and the skin responses (erythema, edema) of different sites for testing drug were observed with naked eyes 0.5 and 1 h before administration and after drug removal respectively every day and recorded. 72 h after the last drug removal, all animals were euthanized, and the skin tissue from the administration site was taken for histopathological examination. The statistical scoring was performed with reference to the skin irritative response scoring standard, and the intensity of skin irritation was evaluated based on the calculated score.

**Table 4 Skin irritation and allergic response scoring standard**

| Irritative response | Score |
|---|---|
| Erythema | |
| No erythema | 0 |
| Mild erythema (barely visible) | 1 |
| Moderate erythema (obviously visible) | 2 |
| Severe erythema | 3 |
| Purple-red erythema to mild eschar formation | 4 |
| Edema | |
| No edema | 0 |
| Mild edema (barely visible) | 1 |
| Moderate edema (obvious protrusion) | 2 |
| Severe edema (skin protrusion of 1 mm with clear outline) | 3 |
| Severe edema (skin protrusion of more than 1 mm with expanding) | 4 |
| Highest total score | 8 |

**Table 5 Evaluation criteria for skin irritation intensity**

| Score | Evaluation |
|---|---|
| 0-0.49 | No irritation |
| 0.5-2.99 | Mild irritation |
| 3.0-5.99 | Moderate irritation |
| 6.0-8.00 | Severe irritation |

Under the conditions of this test, single application administration was performed to the intact skin and injured skin of conventional New Zealand rabbits, and the average irritation scores of the administration sites observed with the naked eye were 0, indicating no irritation. The specific results are shown in Table 6.

Repeated application administration to the intact skin and injured skin of conventional New Zealand rabbits was performed once a day for 14 consecutive days. The average irritation scores of the administration sites observed with the naked eye were all 0, indicating no irritation; the average irritation scores of the histopathological examination were 0, indicating no irritation. The specific results are shown in Table 7.

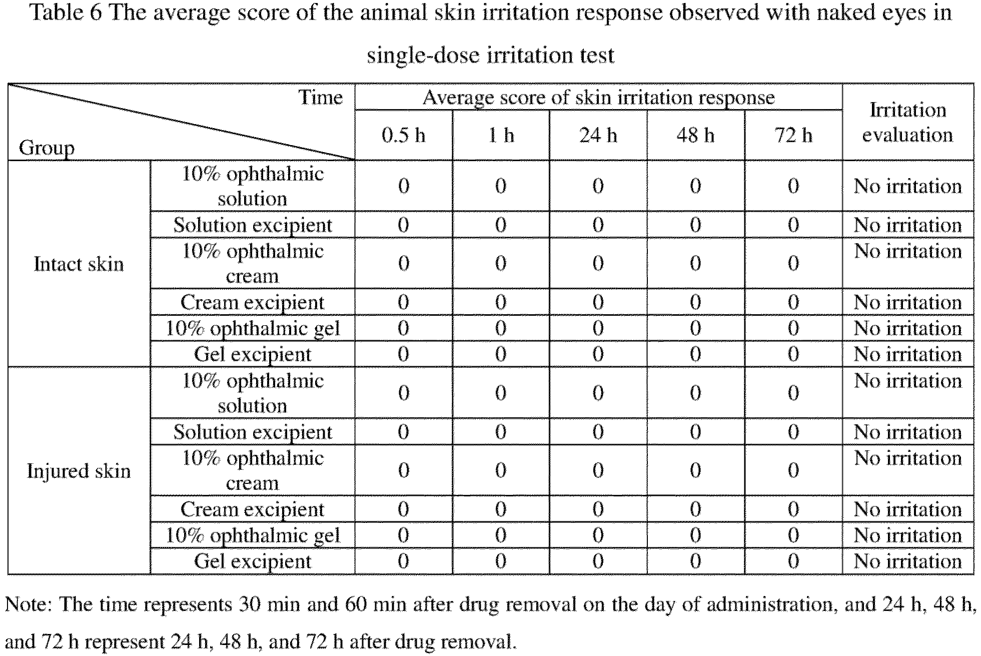

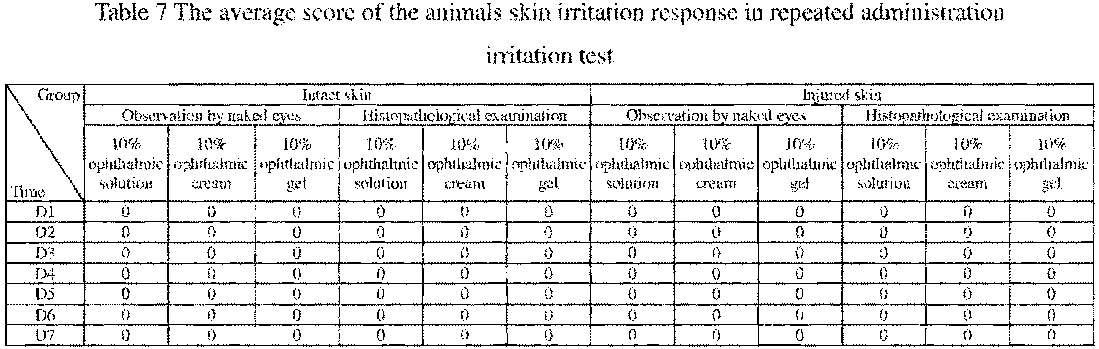

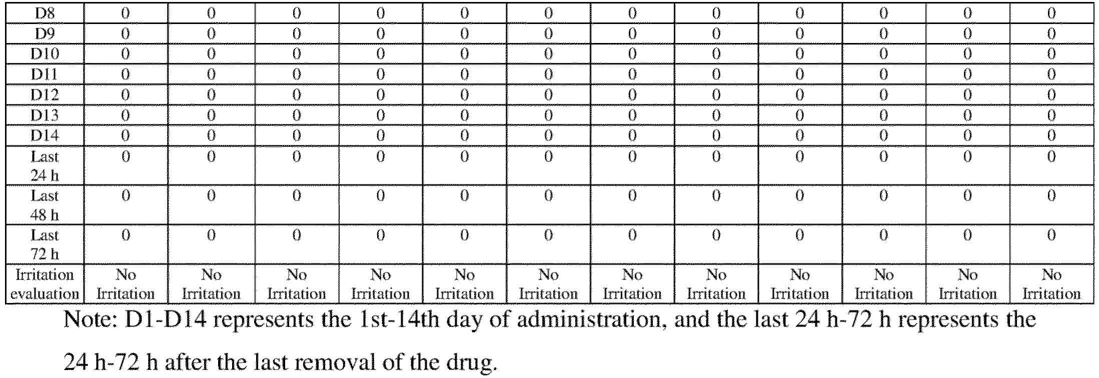

### (2) Skin allergy test

56 healthy guinea pigs were randomly divided into 7 groups, i.e., 10% ophthalmic solution and solution excipient, 10% ophthalmic cream and cream excipient, 10% ophthalmic gel and gel excipient, positive control group, with 8 in each group, half male and half female. Hair removal was performed on the backs of guinea pigs using 10% sodium sulfide solution in ethanol and the hair removal area was about 3.0 cm × 3.0 cm. After 24 h of hair removal, the subjects with no skin injury were screened and selected for the experiments. The administration way was the same as that of the single-dose skin irritation test. In the positive control group, 0.2 ml of 1% 2,4-dinitrochlorobenzene was applied to the hair removal area of each guinea pig for 6 h, and then washed off with warm water. And the same method was used to sensitize again on the 7th and 14th days, for a total of sensitization 3 times. 14 days after the last sensitization of the test substances, the test substances (positive control group: 0.1% 2,4-dinitrochlorobenzene) were applied to the hair removal areas of the corresponding group of guinea pigs again. After 6 h, the tested sites were washed with warm water and the skin allergic response was observed immediately and recorded once 24, 48, and 72 h after washing the test substances, and the allergic response was scored according to the skin allergic response scoring standard (Table 4). The sensitization intensity of the test substances was evaluated based on the sensitization incidence rate, wherein the average score = (erythema formation total score + edema formation total score)/total number of animals, sensitization rate % = (Number of animal cases with erythema, edema, or systemic allergic responses/total number of tested animals) × 100%.

**Table 8 Sensitization intensity**

| Sensitization rate | Grading | Sensitization intensity |
|---|---|---|
| 0-8 | I | Weak sensitization |
| 9-28 | II | Mild sensitization |
| 29-64 | III | Moderate sensitization |
| 65-80 | IV | Strong sensitization |
| 81-100 | V | Extreme sensitization |

During the test, no obvious abnormalities were generally observed in all animals. In the 8 cases in the positive control group (8/8), symptoms of mild to severe erythema and edema can be seen at the administration sites 1 and 24 h after each sensitization and drug removal for the animals, and from the 4th day of sensitization to the 7th day after the last sensitization, white to dark red scabs of varying sizes can be seen at the administration sites of the animals. The sensitization incidence rate was 100%, the grade was level V, and the sensitization intensity was extreme sensitization.

For each group of 10% ophthalmic solution group and solution excipient group, 10% ophthalmic cream group and cream excipient group, 10% ophthalmic gel group and gel excipient group: 1, 24, and 48 h after challenge administration and drug removal, no symptoms of allergic response were seen on the skin of the 8 animals (8/8) at the administration sites. The average score was 0, the sensitization incidence rate was 0%, and the grade was level I, and the sensitization intensity was weak sensitization. The specific results are shown in Table 9.

**Table 9 Statistical table of skin allergic response scores after test challenge and drug removal**

| Group | Time point | Erythema | Edema | Total score | Average score | Number of allergic animals | Sensitization rate (%) | Sensitization intensity |
|---|---|---|---|---|---|---|---|---|
| Positive control group | 1 h | 18 | 4 | 22 | 2.2 | 10 | 100 | Extreme sensitization |
| | 24 h | 15 | 2 | 17 | 1.7 | 10 | 100 | Extreme sensitization |
| | 48 h | 10 | 0 | 10 | 1.0 | 10 | 100 | Extreme sensitization |
| 10% ophthalmic solution group | 1 h | 0 | 0 | 0 | 0 | 0 | 0 | Weak sensitization |
| | 24 h | 0 | 0 | 0 | 0 | 0 | 0 | Weak sensitization |
| | 48 h | 0 | 0 | 0 | 0 | 0 | 0 | Weak sensitization |
| Solution excipient group | 1 h | 0 | 0 | 0 | 0 | 0 | 0 | Weak sensitization |
| | 24 h | 0 | 0 | 0 | 0 | 0 | 0 | Weak sensitization |
| | 48 h | 0 | 0 | 0 | 0 | 0 | 0 | Weak sensitization |
| 10% ophthalmic cream group | 1 h | 0 | 0 | 0 | 0 | 0 | 0 | Weak sensitization |
| | 24 h | 0 | 0 | 0 | 0 | 0 | 0 | Weak sensitization |
| | 48 h | 0 | 0 | 0 | 0 | 0 | 0 | Weak sensitization |
| Cream excipient group | 1 h | 0 | 0 | 0 | 0 | 0 | 0 | Weak sensitization |
| | 24 h | 0 | 0 | 0 | 0 | 0 | 0 | Weak sensitization |
| | 48 h | 0 | 0 | 0 | 0 | 0 | 0 | Weak sensitization |
| 10% ophthalmic gel group | 1 h | 0 | 0 | 0 | 0 | 0 | 0 | Weak sensitization |
| | 24 h | 0 | 0 | 0 | 0 | 0 | 0 | Weak sensitization |
| | 48 h | 0 | 0 | 0 | 0 | 0 | 0 | Weak sensitization |
| Gel excipient group | 1 h | 0 | 0 | 0 | 0 | 0 | 0 | Weak sensitization |
| | 24 h | 0 | 0 | 0 | 0 | 0 | 0 | Weak sensitization |
| | 48 h | 0 | 0 | 0 | 0 | 0 | 0 | Weak sensitization |

### Example 3: Test on the effectiveness of relieving pinkeye

### (1) Test for treating pinkeye with severe conditions

Test group: 24 patients with comparable conditions were selected, 13 males and 11 females, aged 8-75 years old, with severe conditions (the patients had redness in both eyes, redness and swelling of eyelids, photophobia, eye itching, eye burning sensation, lacrimation or epiphora, and more gums in eyes). The 0.05% ophthalmic solution prepared in Example 1 was dripped into the eyes 3 times a day for 5 consecutive days.

Control group: 23 patients with comparable conditions were selected, 12 males and 11 females, aged 8-73 years old, with severe conditions (the patients had redness in both eyes, redness and swelling of eyelids, photophobia, eye itching, eye burning sensation, lacrimation or epiphora, and more gums in eyes). The 0.25% chloramphenicol eye drops was dripped into the eyes 3 times a day for 5 consecutive days.

After 5 days, the therapeutic effect was observed, and a follow-up visit was conducted 15 days later to observe whether there was recurrence. The test groups were compared to the control group, and the cure rate and recurrence rate are shown in Table 10.

**Table 10 Cure rate and recurrence rate of test group and control group**

| | Test group | Control group |
|---|---|---|
| Cure rate | 96% (12 male cases, 11 female cases) | 87% (10 male cases, 10 female cases) |
| Recurrence rate | 4% (1 male case) | 13% (2 male cases, 1 female case) |

The results showed that: for pinkeye with severe conditions, the cure rate of the ophthalmic solution of Example 1 was significantly higher than that of 0.25% chloramphenicol eye drops, and the recurrence rate was significantly lower than that of 0.25% chloramphenicol eye drops.

### (2) Test for treating pinkeye with moderate conditions

Test group: 25 patients with comparable conditions were selected, 13 males and 12 females, aged 8-75 years old, with moderate conditions (the patients had redness in both eyes, redness and swelling of eyelids, photophobia, eye itching, and eye burning sensation). The 0.05% ophthalmic solution prepared in Example 1 was dripped into the eyes 3 times a day for 5 consecutive days.

Control group: 24 patients with comparable conditions were selected, 12 males and 12 females, aged 8-73 years old, with moderate conditions (the patients had redness in both eyes, redness and swelling of eyelids, photophobia, eye itching, and eye burning sensation). The 0.25% chloramphenicol eye drops was dripped into the eyes 3 times a day for 5 consecutive days.

After 5 days, the therapeutic effect was observed, and a follow-up visit was conducted 15 days later to observe whether there was recurrence. The test group was compared to the control group, and the cure rate and recurrence rate are shown in Table 11.

**Table 11 Cure rate and recurrence rate of test groups and control group**

| | Test group | Control group |
|---|---|---|
| Cure rate | 100% (13 male cases, 12 female cases) | 88% (12 male cases, 12 female cases) |
| Recurrence rate | 0% | 12% (2 male cases, 1 female case) |

The results showed that: for pinkeye with moderate conditions, the cure rate of the ophthalmic solution of Example 1 was significantly higher than that of 0.25% chloramphenicol eye drops, and the recurrence rate was significantly lower than that of 0.25% chloramphenicol eye drops.

In summary, the composition containing alanyl-glutamine can effectively treat, relieve or alleviate pinkeye.

### Example 4: Test on the effectiveness of relieving ocular congestion

### (1) Ocular congestion caused by cataract surgery

Test group: 19 patients, whose scores for conjunctival congestion after cataract surgery were all (+++), were selected; wherein there were 7 males and 12 females, aged 62-77 years old. The 0.05% ophthalmic solution prepared in Example 1 was dripped into the eyes at 1 drop/eye/time at an interval of 4 h for each administration, 3 administrations/day for 3 consecutive days.

Control group: 17 patients, whose scores for conjunctival congestion after cataract surgery were all (+++), were selected; wherein there were 6 males and 11 females, aged 62-77 years old. The 0.1% diclofenac sodium eye drops was administered at 1 drop/eye/time at an interval of 4 h for each administration, 3 administrations/day for 3 consecutive days.

The conjunctival congestion of the two groups of patients was observed 30 min after the second administration by eye drop on the third day. The grading standard of conjunctival congestion is divided into: ① No congestion (-); ② Mild congestion (+): the congestion is limited to the palpebral fissure, and the blood vessels are bright red; ③Moderate congestion (++): the congestion can obviously reach the fornix, and the blood vessels are congested and appear dark red; ④Severe congestion (+++): the entire conjunctiva is diffusely congested, and the blood vessels are purple-red and blurred.

The results of the comparison of conjunctival congestion between the two groups of patients are shown in Table 12. The M-W U test of two groups of independent samples was used for statistics, wherein the Mann-Whitney U value was 97.000, P=0.042<0.05; The results showed that the ophthalmic solution of Example 1 was significantly better than the 0.1% diclofenac sodium eye drops in relieving ocular congestion caused by cataract surgery, and the ophthalmic solution of Example 1 had significant clinical application value after cataract surgery.

**Table 12 Comparison of therapeutic effects on ocular congestion in patients after cataract surgery**

| | Number of cases | (+++) | (++) | (+) | (-) |
|---|---|---|---|---|---|
| Test group | 19 | 1 | 1 | 6 | 11 |
| Control group | 17 | 1 | 5 | 7 | 4 |

### (2) Ocular congestion caused by other types of diseases

Patients, whose scores of conjunctival congestion caused by reasons such as immune ocular surface diseases and inflammation were (+++), were selected.

Test group: there were 23 cases, 12 males and 11 females, aged 8-65 years old. The 0.05% ophthalmic solution prepared in Example 1 was dripped into the eyes at 1 drop/eye/time at an interval of 4 h for each administration, 3 administrations/day for 3 consecutive days.

Control group: there were 24 cases, 13 males and 11 females, aged 8-63 years old, and the 0.1% pranoprofen eye drops was dripped into the eyes at 1 drop/eye/time at an interval of 4 h for each administration, 3 administrations/day for 3 consecutive days.

The conjunctival congestion of the two groups of patients was observed 30 min after the second administration by eye drop on the third day. The grading standard of conjunctival congestion is divided into: ① No congestion (-); ② Mild congestion (+): the congestion is limited to the palpebral fissure, and the blood vessels are bright red; ③Moderate congestion (++): the congestion can obviously reach the fornix, and the blood vessels are congested and appear dark red; ④Severe congestion (+++): the entire conjunctiva is diffusely congested, and the blood vessels are purple-red and blurred.

The results of the comparison of conjunctival congestion between the two groups of patients are shown in Table 13. The M-W U test of two groups of independent samples was used for statistics, wherein the Mann-Whitney U value was 182.500, P=0.031<0.05; The results showed that the ophthalmic solution of Example 1 had a significantly better therapeutic effect on ocular congestion caused by other diseases than 0.1% pranoprofen eye drops, and the ophthalmic solution of Example 1 also had significant clinical application value on ocular congestion caused by other types of diseases.

**Table 13 Comparison of the therapeutic effect of ocular congestion caused by other diseases**

| | Number of cases | (+++) | (++) | (+) | (-) |
|---|---|---|---|---|---|
| Test group | 23 | 0 | 2 | 7 | 14 |
| Control group | 24 | 2 | 5 | 9 | 8 |

### (3) Ocular congestion caused by lifestyle or eye makeup

Subjects with ordinary mild ocular congestion or mild ocular congestion without obvious pathogenic factors, or mild ocular congestion caused by eye makeup were recruited (if the patients had mild congestion in both eyes, then the right eye of each patient was selected as the eye for observation).

Test group: there were 60 cases, 15 males and 45 females, aged 20-50 years old. The 0.05% ophthalmic solution prepared in Example 1 was dripped into the eyes at 1 drop/eye/time at an interval of 4 h for each administration, 3 administrations/day for 3 consecutive days.

Control group: there were 60 cases, 16 males and 44 females, aged 19-50 years old, and the sodium hyaluronate eye drops was dripped into the eyes at 1 drop/eye/time at an interval of 4 h for each administration, 3 administrations/day for 3 consecutive days.

The conjunctival congestion of the two groups of subjects was observed 10 and 30 min after the 1st administration by eye drop on the 1st day, and 30 min after the 1st administration by eye drop on the 2nd and 3rd days. The grading standard of conjunctival congestion is divided into: ① No congestion (-); ② Mild congestion (+): the congestion is limited to the palpebral fissure, and the blood vessels are bright red; ③Moderate congestion (++): the congestion can obviously reach the fornix, and the blood vessels are congested and appear dark red; ④Severe congestion (+++): the entire conjunctiva is diffusely congested, and the blood vessels are purple-red and blurred.

The results of the comparison of conjunctival congestion between the two groups of patients are shown in Table 14. 10 and 30 min after the 1st administration, the effectiveness rates of the test group were 65% and 86.3% respectively, and the effectiveness rates of the control group were 23.3% and 28.3% respectively. There was a significant difference in the effectiveness rates between the two groups (P<0.05 or P<0.01). 30 min after the administration on the 2nd and 3rd days, the effectiveness rates of the test group were 98.3% and 98.3% respectively, and the effectiveness rates of the control group were 45% and 55% respectively. There was a significant difference in the effectiveness rates between the two groups (P<0.01). It was suggested that the ophthalmic solution of Example 1 has a significant effect and significant clinical application value in the treatment of ordinary mild ocular congestion or mild ocular congestion without obvious pathogenic factors, or mild ocular congestion caused by eye makeup.

**Table 14 Ocular congestion caused by lifestyle or eye makeup ( x̅ ± s )**

| Degree ee Number of cases | Test group | | | Control group | | |
|---|---|---|---|---|---|---|
| | Before administration | The 1st time-10 min | The 1st time-30 min | Before administration | The 1st time-10 min | The 1st time-30 min |
| (+++) | 11 | 5 | 3 | 10 | 8 | 9 |
| (++) | 29 | 16 | 12 | 27 | 20 | 18 |
| (+) | 20 | 20 | 19 | 23 | 27 | 22 |
| (-) | 0 | 19 | 26 | 0 | 5 | 11 |
| Effectiveness rate | - | 65%* | 86.3%** | - | 23.3% | 28.3% |

| | | | | | | |
|---|---|---|---|---|---|---|
| **P*<0.05, ***P*<0.01, compared to the control group at the same evaluation time point. | | | | | | |

**Continued Table 14 Ocular congestion caused by lifestyle or eye makeup ( x̅ ± s )**

| Degree | Test group | | Control group | |
|---|---|---|---|---|
| | Day 2-30 min | Day 3-30 min | Day 2-30 min | Day 3-30 min |
| (+++) | 0 | 0 | 7 | 5 |
| (++) | 8 | 6 | 18 | 16 |
| (+) | 16 | 10 | 20 | 22 |
| (-) | 36 | 44 | 15 | 17 |
| Effectiveness rate | 98.3%** | 98.3%** | 45% | 55% |

| | | | | |
|---|---|---|---|---|
| **P*<0.05, ***P*<0.01, compared to the control group at the same evaluation time point. | | | | |

### Example 5: Test on the effectiveness of treating corneal ulcers

### The vitro model of corneal ulcer

The rabbit corneas were taken and cut into pieces, 5-10 times volume of 5% triton solution was added and homogenized until there were no lumpy particles, then centrifuged at 3000 r/min for 10 min, and the supernatant was taken. 5 ml of the supernatant was taken and mixed with 5 ml of complete Freund's adjuvant to prepare a mixed solution for later use.

SPF grade ICR mice were divided into normal group, immune group, and cyclophosphamide group, 10 mice/group. The normal group received subcutaneous injection of 0.5 ml of physiological saline per mouse; Each mouse in the immune group and cyclophosphamide group was subcutaneously injected with the mixed solution + physiological saline, the mixed solution + cyclophosphamide 300 mg/kg respectively, and the injection sites were not overlapped; Injection was performed once every other day for a total of 10 times. 3 days after the last injection, the mice were euthanized using the bloodletting method, and the eyeballs were removed and cut into pieces under sterile conditions. At the same time, the cornea and the leukocytes separated from autologous blood were co-cultured in a 24-well culture plate. The cornea in the normal group, cornea in the immune group, cornea in the cyclophosphamide group, and administration to cornea in immune group (0.05% ophthalmic solution described in Example 1) were set, 5 replicate wells were set for each cornea, the solutions were changed once every 24 h, and the growth of corneal tissue cells was observed.

### Test results

The growth rates of the corneal cells at 72 h and 120 h in the normal group were 24.0% and 36.0%, while the growth rates of the corneal cells in the immune group were both 0%. After administration of 0.05% ophthalmic solution, the growth rates of the corneal cells at 72 h and 120 h were 8% and 14%, there was a significant difference (P<0.01) compared to the immune group, thus proving that the ophthalmic solution inhibited the immune response of cell growth and promoted the growth of corneal cells. Therefore, the composition comprising alanyl-glutamine can effectively treat, relieve or alleviate corneal epithelial injury, corneal ulcer, recurrent corneal epithelial erosion, and diabetic keratopathy.

**Table 15 Growth of corneal cells in culture in vitro**

| Group | Calculation parameters | Grow time | |
|---|---|---|---|
| | | 72 h | 120 h |
| Cornea in normal group | Number | 12 | 18 |
| | Percentage% | 24.0** | 36.0** |
| Cornea in immune group | Number | 0 | 0 |
| | Percentage% | 0 | 0 |
| Cornea in cyclophosphamide group | Number | 8 | 11 |
| | Percentage% | 16.0** | 22.0** |
| Administration to cornea in immune group | Number | 4 | 7 |
| | Percentage% | 8.0** | 14.0** |

### Example 6: Test on the effectiveness of relieving asthenopia

### (1) Test for alleviating symptoms of asthenopia

Half an hour after using of the 0.05% ophthalmic cream, 0.05% ophthalmic gel, 0.05% ophthalmic solution prepared in Example 1 of the present disclosure and Zhenshiming eye drops, the alleviation of the symptoms of asthenopia was observed and scored by experienced doctors, and the total effectiveness rate was calculated. The calculation formula is as follows: Improvement rate of symptom score = (pre-treatment score - post-treatment score)/pre-treatment score × 100%
Significantly effective: the improvement rate of symptom score is greater than 80%;
Effective: the improvement rate of symptom score is greater than 40%;
Invalid: the improvement rate of symptom score is less than 40 or has no change or even get worse; Total effectiveness rate = number of people of (significantly effective + effective) / total number of people × 100%

The total number of people in the experiments was 107, including 26 people in the 0.05% ophthalmic cream group, 28 people in the 0.05% ophthalmic gel group, 28 people in the 0.05% ophthalmic solution group, and 25 people in the Zhenshiming eye drops group. The specific results are shown in Table 16.

**Table 16 Alleviation of symptoms of asthenopia**

| | | 0.05% ophthalmic cream (human) | 0.05% ophthalmic gel (human) | 0.05% ophthalmic solution (human) | Zhenshuiming eye drops (Human) |
|---|---|---|---|---|---|
| Alleviation of sore eyes | Invalid | 0 | 1 | 1 | 5 |
| | Effective | 8 | 7 | 9 | 8 |
| | Significantly effective | 18 | 20 | 18 | 12 |
| Alleviation of red bloodshot eyes | Invalid | 1 | 1 | 0 | 6 |
| | Effective | 8 | 9 | 9 | 9 |
| | Significantly effective | 17 | 18 | 19 | 10 |
| Alleviation of dry eyes | Invalid | 0 | 0 | 0 | 4 |
| | Effective | 7 | 7 | 10 | 8 |
| | Significantly effective | 19 | 21 | 18 | 13 |
| Alleviation of eye swelling | Invalid | 4 | 4 | 3 | 5 |
| | Effective | 7 | 6 | 9 | 5 |
| | Significantly effective | 15 | 18 | 16 | 15 |
| Alleviation of blurred vision | Invalid | 4 | 3 | 2 | 6 |
| | Effective | 7 | 7 | 10 | 5 |
| | Significantly effective | 15 | 18 | 16 | 14 |
| Comfort of use | not good | 2 | 1 | 2 | 3 |
| | Moderate | 4 | 4 | 5 | 10 |
| | Good | 20 | 23 | 21 | 12 |
| Total effectiveness (%) | | 92.9 | 94.0 | 95.2 | 80.6 |

It can be seen from the above experimental results that the ophthalmic cream, ophthalmic gel and ophthalmic solution of Example 1 are significantly better than Zhenshiming eye drops in alleviating the symptoms of asthenopia.

### (2) Test of duration of photopic vision

The duration of photopic vision was measured with reference to the "Method for Assessment of Asthenopia Relief Function" in the "Notice on the Issuance of 9 Health Function Assessment Methods including the Method for Assessment of Antioxidant Function, Health Food and Cosmetics Supervision Department, State Food and Drug Administration (2012) No. 107". The study adopted the research method of comparing before and after self-control treatment, and the results are shown in Table 17.

**Table 17 Duration of photopic vision of test group and control group**

| Composition | Duration of photopic vision | | |
|---|---|---|---|
| | Before treatment% | After treatment% | Increase rate % |
| Commercially available eye patches | 43.28±10.69 | 50.43±13.53* | 16.52% |
| 0.05% ophthalmic cream | 48.68±6.37 | 61.26±10.22* | 25.84 |
| 0.05% ophthalmic gel | 50.75±7.19 | 61.55±11.28* | 23.25 |
| 0.05% ophthalmic solution | 45.39+5.82 | 58.05±9.61* | 27.89 |

| | | | |
|---|---|---|---|
| Note: *P<0.05 | | | |

Table 17 shows that: after using the ophthalmic cream, ophthalmic gel and ophthalmic solution of Example 1, the duration of photopic vision was significantly improved (p<0.05) compared to that before the self-control test, and the increase rates were all greater than 20%, and the effect was significantly better than Zhenshiming eye drops.

Combined with the significant effects of the ophthalmic cream, ophthalmic gel and ophthalmic solution in alleviating symptoms of asthenopia and the duration of photopic vision, it was effectively proved that the composition comprising alanyl-glutamine had the function of alleviating asthenopia.

### Example 7: Study on the therapeutic effect of ocular discomfort mainly caused by foreign body sensation

The foreign body sensation score was used as the judgment standard, a score of 0 indicated no foreign body sensation and a score of 10 indicated strong foreign body sensation. A total of 27 patients were selected who had a self-scoring of foreign body sensation higher than 6 scores after surgery for eye diseases such as cataract or glaucoma.

Test group: there were 14 cases, 8 males and 6 females, aged 63-72 years old. The 0.05% ophthalmic solution prepared in Example 1 was dripped into the eyes at 1 drop/eye/time at an interval of 4 h for each administration, 3 administrations/day for 7 consecutive days.

Control group: there were 13 cases; 6 males and 7 females, aged 63-73 years old. The sodium hyaluronate eye drops was administered at 1 drop/eye/time at an interval of 4 h for each administration, 3 administrations/day for 7 consecutive days.

The self-scoring of the foreign body sensation of the two groups of patients was observed after administration.

Results: The results of the comparison of foreign body sensation between the two groups of patients after drug administration are shown in Table 18. T-test of two groups of independent samples was used for statistics. Compared to the control group, i.e., compared between groups, the foreign body sensation scores of patients in the test group were significantly reduced at 1 h, 3 days, and 7 days after administration (P<0.05 or P<0.01); The foreign body sensation scores of patients in the test group was significantly reduced at 1 h, 3 days, and 7 days after administration (P<0.05 or P<0.01) compared to those before administration, i.e., compared within the group, and the foreign body sensation of patients of the test group was reduced more obviously along with the increase of the times of administration. The results showed that the ophthalmic solution of the present disclosure can reduce the postoperative ocular discomfort mainly manifested by foreign body sensation, and can promote the postoperative wound healing.

**Table 18 Comparative analysis of foreign body sensation of eyeball after surgery ( x̅ ± s )**

| Group | n | Before administratio n | 1 h after the first administration | Administer for 3 days | Administration for 7 days |
|---|---|---|---|---|---|
| Test group | 14 | 7.57±1.05 | 4.71±0.70*# | 2.64±0.89**## | 0.86±0.91**## |
| Control group | 13 | 7.54±1.15 | 6.08±1.44 | 5.77±0.80 | 4.76±0.89# |

| | | | | | |
|---|---|---|---|---|---|
| Compared to the control group, *P<0.05, ***P*<0.01; Compared to before administration, #P<0.05, ##P<0.01. | | | | | |

### Example 8: Test for treating, alleviating, or relieving ocular surface disease caused by meibomian gland dysfunction (MGD)

90 patients diagnosed with moderate or severe MGD in the ophthalmology clinic were selected (if the patients had moderate or severe MGD in both eyes, then the right eye of each patient was selected as the eye for observation). All patients were randomly divided into control group and study group, with 45 cases in each group.

All patients were given basic treatment, and the specific operation methods are: ①Cleaning of the palpebral margin part: a medical cotton swab was soaked in 0.9% sodium chloride injection, and the cotton swab was used to clean the palpebral margin once a day. ②Hot compress: the eye pad of greenhouse mirror was worn, the elastic band was adjusted until comfortable, the power was turned on, and mode 2 was selected (negative pressure massage + 40°C circular hot compress), 15 min/time, once/day.

Test group: there were 23 males and 22 females, with an average age of (37.6±7.1) years. The 0.05% ophthalmic solution prepared in Example 1 was dripped into the eyes at 1 drop/eye/time at an interval of 4 h for each administration, 3 administrations/day for 7 consecutive days.

Control group: there were 24 males and 21 females, with an average age of (36.9±7.0) years. and the polyvinyl alcohol eye drops was dripped into the eyes at 1 drop/eye/time at an interval of 4 h for each administration, 3 administrations/day for 7 consecutive days.

Degree of congestion of palpebral conjunctiva: diffusion illumination method under slit lamp microscope was used for observation. 0 score, there is no congestion or slight congestion in the conjunctiva at the palpebral margin, and there is no telangiectasia at the opening of the meibomian glands; 1 point, there is congestion in the conjunctiva at the palpebral margin, but the congestion is limited to the fornix, and the blood vessels are bright red, and there is no telangiectasia at the opening of the meibomian glands; 2 points, there is congestion in the conjunctiva at the palpebral margin, the blood vessels are dark red and have unclear course, and there is telangiectasia at the opening of the meibomian glands, and the range is <1/2 of the palpebral margin; 3 points: there is diffuse congestion in the conjunctiva at the palpebral margin, the blood vessels are purple-red, the tarsal texture is blurred, and there is telangiectasia at the opening of the meibomian glands, and the range is ≥ 1/2 of the palpebral margin.

The results of conjunctival congestion in the two groups of patients are shown in Table 19. 7 days after the administration for treatment, the conjunctival congestion scores of the patients in the test group were significantly reduced compared to those before the treatment (P <0.05), but the difference in conjunctival congestion scores of the control group was small (P>0.05); After treatment, the conjunctival congestion scores of the patients in the test group were significantly reduced (P<0.05) compared to the control group. The above results suggested that the ophthalmic solution of Example 1 can significantly alleviate palpebral conjunctiva congestion in patients with MGD.

**Table 19 Conjunctival congestion in patients with palpebral conjunctival dysfunction ( x̅ ± s )**

| Group | Before treatment | After treatment |
|---|---|---|
| Control group | 1.97±0.75 | 1.41±0.72 |
| Test group | 2.06±0.81 | 0.91±0.74*# |

| | | |
|---|---|---|
| *P<0.05, compared to control group; #P<0.05, compared to before administration. | | |

Although the specific embodiments have been described, for the applicant or a person skilled in the art, the substitutions, modifications, changes, improvements, and substantial equivalents of the above embodiments may exist or cannot be foreseen currently. Therefore, the submitted appended claims and claims that may be modified are intended to cover all such substitutions, modifications, changes, improvements, and substantial equivalents.

## Claims

1. Use of alanyl-glutamine in the manufacture of a drug for treating, relieving or alleviating ocular surface diseases and/or conditions of non-dry eyes.

2. Use of alanyl-glutamine in the manufacture of a drug for treating, relieving or alleviating ocular surface diseases and/or conditions associated with corneal epithelial injury.

3. Use of alanyl-glutamine in the manufacture of a drug for treating, relieving or alleviating ocular surface diseases and/or conditions caused by diabetes.

4. Use of alanyl-glutamine in the manufacture of a drug for treating, relieving or alleviating ocular surface diseases and/or conditions associated with keratopathy caused by diabetes.

5. Use of alanyl-glutamine in the manufacture of a drug for treating, relieving or alleviating ocular surface diseases and/or conditions associated with recurrent corneal epithelial erosion.

6. Use of alanyl-glutamine in the manufacture of a drug for treating, relieving or alleviating ocular surface diseases and/or conditions associated with surgically related recurrent corneal epithelial erosion.

7. Use of alanyl-glutamine in the manufacture of a drug for treating, relieving or alleviating ocular surface diseases and/or conditions associated with ocular trauma and/or post-surgical tissue repair and healing.

8. Use of alanyl-glutamine in the manufacture of a drug for treating, relieving or alleviating ocular surface diseases and/or conditions associated with conjunctivitis.

9. Use of alanyl-glutamine in the manufacture of a drug for treating, relieving or alleviating ocular surface diseases and/or conditions associated with ocular congestion.

10. Use of alanyl-glutamine in the manufacture of a drug for treating, relieving or alleviating ocular surface diseases and/or conditions associated with meibomian gland dysfunction.

11. Use of alanyl-glutamine in the manufacture of a drug for treating, relieving or alleviating ocular surface diseases and/or conditions associated with asthenopia.

12. Use of alanyl-glutamine in the manufacture of a drug for preventing myopia.

13. Use of alanyl-glutamine in the manufacture of a beauty product, a health-care product for relieving or alleviating asthenopia.

14. Use of alanyl-glutamine in the manufacture of a beauty product, a health-care product for preventing myopia.

15. Use of alanyl-glutamine in the manufacture of a beauty product, a health-care product for eyes.

16. An ophthalmic composition comprising alanyl-glutamine and an ophthalmologically acceptable excipient, wherein the ophthalmic composition is in the form of eye drops, suspensions, gels, eye ointments, emulsions, eye pads, eye patches, eye masks, eye creams, sprays, injections or implants.

17. The ophthalmic composition of claim 16, wherein the ophthalmic composition is in an isotonic or hypotonic form.

18. The ophthalmic composition of claim 16 or 17, wherein the concentration of the alanyl-glutamine in the ophthalmic composition is 0.05% w/w to 10% w/w.

19. The ophthalmic composition of any one of claims 16-18, wherein the ophthalmologically acceptable excipient includes one or more of an osmotic pressure regulator, a bacteriostatic agent, a viscosifier, a pH regulator, a stabilizer, a surfactant, a humectant, etc.

20. A method for treating, relieving or alleviating ocular surface diseases and/or conditions of non-dry eyes, ocular surface diseases and/or conditions associated with corneal epithelial injury, ocular surface diseases and/or conditions caused by diabetes, ocular surface diseases and/or conditions associated with keratopathy caused by diabetes, ocular surface diseases and/or conditions associated with recurrent corneal epithelial erosion, ocular surface diseases and/or conditions associated with surgically related recurrent corneal epithelial erosion, ocular surface diseases and/or conditions associated with ocular trauma and/or post-surgical tissue repair and healing, ocular surface diseases and/or conditions associated with conjunctivitis, ocular surface diseases and/or conditions associated with ocular congestion, ocular surface diseases and/or conditions associated with meibomian gland dysfunction, ocular surface diseases and/or conditions associated with asthenopia, myopia, the method comprises administering to a subject a therapeutically effective amount of alanyl-glutamine.

21. A method for treating, relieving or alleviating ocular surface diseases and/or conditions of non-dry eyes, ocular surface diseases and/or conditions associated with corneal epithelial injury, ocular surface diseases and/or conditions caused by diabetes, ocular surface diseases and/or conditions associated with keratopathy caused by diabetes, ocular surface diseases and/or conditions associated with recurrent corneal epithelial erosion, ocular surface diseases and/or conditions associated with surgically related recurrent corneal epithelial erosion, ocular surface diseases and/or conditions associated with ocular trauma and/or post-surgical tissue repair and healing, ocular surface diseases and/or conditions associated with conjunctivitis, ocular surface diseases and/or conditions associated with ocular congestion, ocular surface diseases and/or conditions associated with meibomian gland dysfunction, ocular surface diseases and/or conditions associated with asthenopia, myopia, the method comprises administering to a subject a therapeutically effective amount of the ophthalmic composition of any one of claims 16-19.
